# EUROPÄISCHE PATENTSCHRIFT

(11) **EP 4 132 442 B1**
(45) Veröffentlichungstag und Bekanntmachung des Hinweises auf die Patenterteilung: **29.05.2024**
(21) Anmeldenummer: 21716427.6
(22) Anmeldetag: 04.04.2021
(51) Int. Cl.: A61F 13/20, A61F 13/26, B65G 47/74, B65G 49/00

(54) **VORRICHTUNG UND VERFARHEN ZUM FÜGEN MEHRTEILIGER TAMPON-APPLIKATOREN**
DEVICE AND METHOD FOR JOINING MULTI-PART TAMPON APPLICATORS
DISPOSITIF ET PROCÉDÉ POUR ASSEMBLER DES APPLICATEURS DE TAMPON EN PLUSIEURS PARTIES

(30) Priorität: 06.04.2020 CH 4122020
(43) Veröffentlichungstag der Anmeldung: 15.02.2023
(73) Patentinhaber: Ruggli AG, 5322 Koblenz (CH)
(72) Erfinder: SCHULER, Samuel, 4051 Basel (CH); SCHEIBER, Patrik, 8155 Niederhasli (CH); ZUDDAS, Antonello, 8212 Neuhausen am Rheinfall (CH)
(74) Vertreter: IPrime Rentsch Kaelin AG
(86) Internationale Anmeldenummer: PCT/EP2021/058822
(87) Internationale Veröffentlichungsnummer: WO 2021/204732

(56) Entgegenhaltungen:
- EP-A1- 2 335 666
- CH-A2- 715 307
- US-A- 2 624 078

## Beschreibung

Die vorliegende Erfindung betrifft eine Vorrichtung zum Fügen mehrteiliger Tampon-Applikatoren, sowie ein Verfahren zum Fügen mehrteiliger Tampon-Applikatoren, beides insbesondere dreiteiliger Tampon-Applikatoren.

### Technologischer Hintergrund

Tampon-Applikatoren sind Einführhilfen für Tampons. In ihrer einfachsten Ausführungsform sind Tampon-Applikatoren zylinderförmige Rohre, in denen ein Tampon platziert wird. Die Tampon-Applikatoren weisen eine distale Mündungsöffnung auf, durch welcher der Tampon nach erfolgtem Platzieren in der Körperöffnung ausgestossen wird. Oft sind Tampon-Applikatoren zu diesem Zweck mit einem entsprechenden Stössel ausgestattet. Auch dieser Stössel kann in der einfachsten Ausführungsform als röhrenförmiger Zylinder, in diesem Fall Innenzylinder, ausgestaltet sein.

Ein derartiger zweiteiliger Tampon-Applikator kann zudem mit einem Auszugmechanismus ausgestattet sein. Bei solchen, meist zweiteiligen, Ausgestaltungen, befindet sich der Innenzylinder zunächst im Wesentlichen innerhalb des Aussenzylinders und kann in manchen Fällen auch zunächst den Tampon beherbergen. Im Betrieb wird der Innenzylinder zunächst durch die proximale Öffnung des Aussenzylinders gezogen, bis eine im Vorfeld gespannte mechanische Greifvorrichtung mit dem Tampon verrastet. Meistens ist am proximalen Ende des Aussenzylinders ein Stopp vorgesehen, sodass der Innenzylinder nicht zu weit hinausgezogen werden kann. Entsteht durch dieses Verrasten eine Greifverbindung zwischen dem Innenzylinder und dem Tampon, kann ein erneutes Hineinpressen des Innenzylinders in den Aussenzylinder den Tampon aus Letzterem verdrängen und durch die Mündungsöffnung in die Körperöffnung entlassen.

Meistens sind Tampon-Applikatoren aus einem Kunststoff gefertigt und verfügen an geeigneter Stelle, z.B. im proximalen Bereich eines Aussenzylinders, über einen Griffbereich. Verbesserte Greifeigenschaften können über eine Aufrauhung des Materials, eine Gummierung, besondere Strukturierung oder eine Griffmulde gewährleistet werden.

Neben der zweiteiligen Ausführung können drei-, oder gar mehrteilige Tampon-Applikatoren den Stössel mindestens teilweise teleskopartig aufbauen, zum Beispiel in dem eine Mehrzahl an Innenzylindern vorgesehen ist, die zunächst gegeneinander gelagert verschiebbar sind, und nach einem gegenseitigen verrasten zu einem starren Stössel werden.

All diese teleskopartigen Aufbauweisen von Tampon-Applikatoren dienen insbesondere dazu, Tampons mit Tampon-Applikatoren insgesamt kompakter auszugestalten.

Um Tampon-Applikatoren bereitzustellen, die sowohl einen geeigneten Griffbereich aufweisen als auch möglichst kompakt sind, haben sich besonders dreiteilige Tampon-Applikatoren bewährt. Diese Tampon-Applikatoren umfassen im Wesentlichen drei röhrenförmige Zylinder. Der Aussenzylinder weist einen konisch verjüngenden Kopfbereich auf, der in eine distale Mündung endet, durch welche der Tampon im Betrieb ausgestossen wird. Der proximale Bereich des Aussenzylinders weist eine Griffmulde, oder einen sich anderweitig verjüngenden Bereich auf, der das Halten des Tampon-Applikators erleichtert, insbesondere dann erleichtert, wenn der Tampon ausgestossen wird, d.h. der Stössel verwendet wird, um den Tampon aus dem Zylinder auszustossen. Der Stössel ist insgesamt zweiteilig untergebracht, wobei in einem Lagerzustand ein erster Innenzylinder im Aussenzylinder gelagert ist und einen zweiten Innenzylinder beherbergt. Um den Tampon-Applikator von einem gelagerten Zustand in einen betriebsbereiten Zustand zu überführen, wird am zweiten Innenzylinder, welcher bevorzugt einen Griffbereich aufweist, gezogen, bis dieser teleskopartig aus dem ersten Innenzylinder ausfährt und mit diesem verrastet, z.B. indem eine entsprechende Nut und/oder ein Flansch am ersten und/oder zweiten Innenzylinder ausgebildet ist. Schliesslich wird auch der zweite Zylinder ein Stück weit aus dem Griffbereich des Aussenzylinders hinausgezogen und tritt in eine Wirkverbindung mit dem Tampon, sodass ein erneutes Einführen des nunmehr verrasteten Stössels aus den beiden Innenzylindern eine Verdrängung des Tampons herbeiführt und diesen aus den Mündungsbereich des Aussenzylinders in die Körperöffnung drückt.

Um die Teile zu fügen, ist es von Vorteil, wenn gewisse Strukturen zunächst reibungslos, d.h. ohne dass ein mechanischer Widerstand überwunden werden muss, ineinander geführt werden. Anschliessend findet ein thermoplastisches Verformen statt, welches den Fügeprozess abschliesst und die einzelnen Applikator-Teile in Längsrichtung verschiebbar gelagert miteinander fügt.

Entscheidend für die Herstellung von Tampon-Applikatoren ist es, bei einer konstant hohen Qualität, die von einem derartigen Hygieneprodukt erwartet wird, möglichst effizient zu produzieren.

CH715307 offenbart eine Vorrichtung zur Bestückung von Tampon-Applikatoren.

Somit besteht ein Bedarf an Vorrichtungen und Maschinen, die geeignet sind, um derartige mehrteilige Tampon-Applikatoren zu fügen, damit sie später mit den entsprechenden Tampons bestückt werden können und der Konfektionierung zugeführt werden können.

Grundsätzlich sind derartige Vorrichtungen lineare Fügestrassen, bei denen schrittweise die verschiedenen Teile ineinandergefügt werden. Dies hat sich jedoch als nachteilig herausgestellt, da der Platzbedarf vergleichsweise hoch ist, und eine Überwachung des Prozesses zur Sicherstellung der Qualität umständlich ist.

### Darstellung der Erfindung

Es ist eine Aufgabe der vorliegenden Erfindung, eine Vorrichtung bereitzustellen, mit welcher mindestens ein Nachteil des Bekannten überwunden wird. Es ist eine besondere Aufgabe der vorliegenden Erfindung, eine Vorrichtung der eingangs genannter Art bereitzustellen, mit der hohe Stückzahlen bei gleichzeitig konstanter Qualität an Tampon-Applikatoren gefügt werden können.

Mindestens eine dieser Aufgaben wurde mit dem kennzeichnenden Teil der unabhängigen Ansprüche gelöst.

Diese Aufgabe wurde mit einer Vorrichtung zum Fügen mehrteiliger Tampon-Applikatoren, insbesondere dreiteiliger Tampon-Applikatoren, und einem entsprechenden Verfahren gemäss kennzeichnenden Teilen der unabhängigen Ansprüche gelöst.

Ein Aspekt der vorliegenden Erfindung betrifft eine Vorrichtung zum Fügen mehrteiliger Tampon-Applikatoren, insbesondere dreiteiliger Tampon-Applikatoren. Die mehrteiligen Tampon-Applikatoren umfassen einen Aussenzylinder und einen ersten Innenzylinder. Die Vorrichtung umfasst mindestens eine Fügestation mit mindestens einem Aufnahmefinger zur Aufnahme je eines Aussenzylinders und eines ersten Innenzylinders. Die erfindungsgemässe Vorrichtung umfasst weiter eine erste Innenzylinderzufuhr zur Bestückung des mindestens einen Aufnahmefingers mit mindestens einem ersten Innenzylinder. Weiter umfasst sie eine Aussenzylinderzufuhr zur Bestückung des mindestens einen Aufnahmefingers mit mindestens einem Aussenzylinder. Eine erste Bearbeitungsstation dient zur Fügung des mindestens einen Aussenzylinders mit dem mindestens einen ersten Innenzylinder. Dabei ist die mindestens eine Fügestation radial auf einer Kreisscheibe angeordnet. Insbesondere ist sie dergestalt auf einer Kreisscheibe angeordnet, dass der mindestens eine Aufnahmefinger im Wesentlichen radial ausgerichtet ist.

Im Sinne der vorliegenden Erfindung kann ein mehrteiliger Tampon-Applikator als ein Tampon-Applikator eingangs genannter Art verstanden werden. Ein solcher Applikator umfasst im Wesentlichen einen Aussenzylinder, der im Betrieb das distale Ende des Tampon-Applikators ausmacht und als distales Ende eine Mündungsöffnung aufweist, durch welche der Tampon ausgestossen wird. Weiter umfasst ein solcher Tampon-Applikator im vorliegenden Beispiel mindestens einen ersten Innenzylinder, der einen Durchmesser aufweist, der geringer ist als der Innendurchmesser des ersten Aussenzylinders, sodass dieser in Längsrichtung des Tampon-Applikators gelagert und bewegbar ist. Besonders bevorzugt ist der erste Innenzylinder in seiner translatorischen Bewegung in Längsrichtung geführt. Dies kann z.B. durch einen weiteren Zylinder geschehen, im vorliegenden Beispiel durch den Aussenzylinder.

Ein erfindungsgemässer Aussenzylinder kann mit beliebigen weiteren Strukturen ausgestattet sein. So kann der Aussenzylinder z.B. im distalen Mündungsbereich blütenblattförmige Strukturen aufweisen, die ein Abschliessen des distalen Mündungsbereichs ermöglichen. Weiter kann der Aussenzylinder mit Noppen, Gummierungen, Aufrauhungen, Einbuchtungen oder anderweitigen Griffmulden versehen sein, welche eine Handhabe des Aussenzylinders und des Tampon-Applikators insgesamt erleichtern. Im vorliegenden Beispiel kann der erfindungsgemässe Tampon-Applikator einen Aussenzylinder aufweisen, der am proximalen Ende verjüngt ist. Dieser verjüngte Bereich kann mit den benannten Strukturen versehen sein, die ein Greifen erleichtern. Im Betrieb würde der Tampon-Applikator an diesem verjüngten Bereich gegriffen werden, eingeführt werden und durch Betätigung eines ersten und/oder zweiten Innenzylinders durch Verschiebung dieses ersten und/oder zweiten Innenzylinders in der Längsachse des Tampon-Applikators zu einem Ausstoss eines Tampons führen, insbesondere durch blütenblattförmige Elemente an der distalen Mündungsöffnung.

Im Sinne der vorliegenden Erfindung findet eine Fügung dann statt, wenn die Teile bezüglich einander in bevorzugt beiden translatorischen Bewegungsrichtungen entlang der Längsachse einen Stopp aufweisen. Dies kann z.B. über einen Flansch geschehen, eine Nut und/oder eine Rippe, welche die Bewegung eines Teils jenseits eines bestimmten Arretierungspunktes unterbindet. Diese Fügung kann bedeuten, dass ein bestimmter Rastpunkt ausgebildet ist, indem z.B. einzelne Teile in eine starre Wirkverbindung zueinander treten können. Zum Beispiel ist ein dreiteiliger Tampon-Applikator denkbar, der aus einem Aussenzylinder, einem ersten Innenzylinder und einem zweiten Innenzylinder besteht. Der Aussenzylinder weist einen grösseren Aussendurchmesser als der erste Innenzylinder auf und der zweite Innenzylinder wiederum einen kleineren Aussendurchmesser als der erste Innenzylinder. Insgesamt sind sowohl der zweite als auch der erste Innenzylinder im Durchmesser derart geringer als der erste Aussenzylinder, dass sie in den Aussenzylinder geschoben werden können. Der erste und der zweite Aussenzylinder bilden in dieser Ausführungsform einen teleskopartigen Stössel zum Ausstoss des Tampons aus dem Aussenzylinder. Dazu wird in einem ersten Schritt der zweite Aussenzylinder in proximaler Richtung aus dem ersten Innenzylinder hinausgezogen, bis er über eine Rastverbindung mit dem ersten Innenzylinder im Wesentlichen starr ist. Weiter wird der erste Innenzylinder soweit aus dem Aussenzylinder hinausgezogen, bis sein distales Ende in Wirkverbindung mit dem distalen Ende eines Tampons gelangt. Wird nun der gesamte Stössel wieder in den Aussenzylinder hineingeschoben, so erzeugt diese Wirkverbindung einen Druck auf das distale Ende des Tampons und schiebt diesen aus dem ersten Aussenzylinder durch die Mündung am distalen Ende des ersten Aussenzylinders hinaus in die Körperöffnung.

Eine Fügung im Sinne der vorliegenden Erfindung würde bei diesem dreiteiligen Tampon-Applikator bestehen, wenn alle drei Teile die oben genannten translatorischen Bewegungen zwar ausführen können, aber verhindert wird, dass der zweite Innenzylinder aus dem ersten Innenzylinder hinausfällt, resp. der erste Innenzylinder aus dem Aussenzylinder hinausfällt. Dazu kann im Innenumfang des Aussenzylinders, resp. des ersten Innenzylinders ein Stopp vorgesehen sein. Zudem kann im Aussenumfang des ersten Innenzylinders und des zweiten Innenzylinders ein entsprechender Flansch vorgesehen sein, der mit diesem Stopp in Wirkverbindung kommt. Die konzentrisch angeordneten Zylinder sind zwar translatorisch zueinander verschiebbar, können allerdings nicht auseinanderfallen. Ein solcher Tampon-Applikator wäre ein erfindungsgemässes Erzeugnis einer entsprechenden Vorrichtung gemäss vorliegender Erfindung. Dieser Tampon-Applikator würde im weiteren Prozessverlauf einer Bestückung mit einem Tampon zugeführt werden können. In einer besonderen Ausführungsform wäre eine solche Fügung im Sinne der vorliegenden Erfindung zum Beispiel mittels einer konischen Aufweitung des proximalen Endes des zweiten Innenzylinders erlangt.

Im Sinne der vorliegenden Erfindung seien die Begriffe distal und proximal in Bezug auf die Anwendung durch den Benutzer zu verstehen. In diesem konkreten Fall würde z.B. das proximale Ende das Ende sein, welches der Hand des Benutzers zugewandt ist. Das distale Ende wäre dabei das in der Nutzung der Körperöffnung zugewandte Ende.

In einer besonderen Ausführungsform ist die mindestens eine Fügestation dergestalt radial auf der Kreisscheibe angeordnet, dass im Betrieb von der Kreisscheibe ein Bearbeitungsumfang abgefahren werden kann, indem die Kreisscheibe um ihren Kreismittelpunkt dreht. Besonders bevorzugt sind ebenfalls radial aussen am Bearbeitungsumfang die erste Innenzylinderzufuhr, die Aussenzylinderzufuhr und die erste Bearbeitungsstation angeordnet.

Im Sinne der vorliegenden Erfindung liegt eine radiale Ausrichtung vor, resp. ist der mindestens eine Aufnahmefinger im Wesentlichen radial ausgerichtet, wenn z.B. der betreffende Aussenfinger betreffend seiner Längsausdehnung im Wesentlichen parallel zu einem Radius der Kreisscheibe angeordnet ist.

Durch die erfindungsgemässe Vorrichtung kann das Fügen mehrteiliger Tampon-Applikatoren effizient anhand einer drehbaren Kreisscheibe stattfinden, die die Fügestation mit den entsprechenden Aufnahmefingern jeweils den Zufuhren und den einzelnen Bearbeitungsstationen, sowie einem Auswurf präsentiert, sodass zwischen diesen und der Fügestation eine Wirkverbindung entstehen kann. Durch die kreisförmige Ausgestaltung ist die gesamte Vorrichtung platzsparend und kann mit hohen Stückzahlen operieren.

In einer besonderen Ausführungsform sind eine Mehrzahl an Fügestationen radial auf der Kreisscheibe vorgesehen. Besonders bevorzugt sind die Fügestationen im Wesentlichen radialsymmetrisch auf der Kreisscheibe angeordnet, sodass beim Drehen der Kreisscheibe Unwuchten weitgehend vermieden werden.

In einer weiteren besonderen Ausführungsform umfasst eine erfindungsgemässe Fügestation eine Mehrzahl an Aufnahmefingern. Somit kann eine erfindungsgemässe Vorrichtung chargenweise fügen, indem ein Batch an Tampon-Applikatoren in Abhängigkeit der Anzahl Aufnahmefinger auf einer Fügestation zeitgleich einer Bearbeitungsstation zugeordnet werden kann.

In einer besonderen Ausführungsform umfasst der mehrteilige Tampon-Applikator einen zweiten Innenzylinder. Die Vorrichtung umfasst dann weiter eine zweite Innenzylinderzufuhr zur Bestückung des mindestens einen Aufnahmefingers mit mindestens einem zweiten Innenzylinder. Zudem umfasst die Vorrichtung weiter eine zweite Bearbeitungsstation zur Fügung des mindestens einen ersten Innenzylinders mit dem mindestens einen zweiten Innenzylinder. In dieser Ausführungsform dient die Vorrichtung dem Fügen von dreiteiligen Tampon-Applikatoren.

In einer besonderen Ausführungsform ist die erste Bearbeitungsstation ausgelegt, um den ersten oder zweiten Innenzylinder umzuformen.

In einer besonderen Ausführungsform ist die zweite Bearbeitungsstation ausgestattet, um einen ersten oder zweiten Innenzylinder umzuformen.

In einer besonderen Ausführungsform umfasst die vorliegende Erfindung eine Mehrzahl am Bearbeitungsstationen, die jeweils ausgelegt sind, stets einen proximalen Innenzylinder umzuformen. So wäre bei es beim Beispiel eines dreiteiligen Tampon-Applikators stets der zweite Innenzylinder, der umgeformt würde. Jede Bearbeitungsstation kann in einer weiteren Ausführungsform ausgelegt sein, den zweiten Innenzylinder, um ein bestimmtes Mass umzuformen. So kann das Umformen des zweiten Innenzylinders schonend von statten gehen, was insgesamt zu einer besseren Umformung führt.

In einer besonderen Ausführungsform ist die Bearbeitungsstation ausgelegt eine konische Aufweitung als Umformung vorzunehmen.

In einer besonderen Ausführungsform umfasst der mindestens eine Aufnahmefinger eine Mehrzahl an Durchmessern. Insbesondere weist der Aufnahmefinger sich in radialer Richtung verjüngende Durchmesser auf.

In einer besonderen Ausführungsform ist jeder Durchmesser ausgelegt, um jeweils einen Aussenzylinder, einen ersten Innenzylinder und/oder einen zweiten Innenzylinder aufzunehmen.

Entsprechend von innen nach aussen, d.h. in radialer Richtung der Kreisscheibe, wären dies ein erster Durchmesser, welcher geeignet ist, einen Aussenzylinder aufzunehmen, ein zweiter Durchmesser, welcher geeignet ist, einen ersten Innenzylinder aufzunehmen, und ein dritter Durchmesser, welcher geeignet ist, einen zweiten Innenzylinder aufzunehmen.

Im vorliegenden Fall sind alle Zylinder als Hohlzylinder ausgestaltet, welche ein Lumen umfassen. Dieses Lumen wird entsprechend vom Durchmesser des Aufnahmefingers im Wesentlichen gefüllt, wenn der Aufnahmefinger den entsprechenden Zylinder aufnimmt.

Im Betrieb würde ein Bestücken eines Aufnahmefingers von innen nach aussen erfolgen, d.h. z.B., es würde ein zweiter Innenzylinder bei einem dreiteiligen Tampon-Applikator zunächst auf den kleinsten Durchmesser des Aufnahmefingers aufgebracht. Anschliessend würde über diesen zweiten Innenzylinder ein erster Innenzylinder gestülpt und entgegen der radialen Richtung über den entsprechenden mittleren Durchmesser des Aufnahmefingers geführt. Zuletzt würde ein Aussenzylinder über beide Innenzylinder gestülpt bis zu einem entsprechenden Durchmesser des Aufnahmefingers.

In einer besonderen Ausführungsform weist der Aufnahmefinger eine Mehrzahl an Schultern auf, welche als Stopp dienen, um den entsprechenden Durchmesser zur Aufnahme eines Zylinders zu definieren.

In einer weiteren besonderen Ausführungsform weist der Aufnahmefinger eine sich verjüngende Spitze auf. Diese Spitze kann z.B. konisch zulaufend ausgestaltet sein, um die Aufnahme und Führung der Zylinder zu erleichtern.

In einer besonderen Ausführungsform sind die Durchmesser durch eine Schulter als Stopp gegeneinander begrenzt, welche in eine konische Verjüngung übergehen, die in den kleineren Durchmesser überführt.

Die Längen der verschiedenen Durchmesserbereiche des Aufnahmefingers betragen zwischen einem Drittel und 80 % der Längen der entsprechenden zweiten Innenzylinder.

In einer besonderen Ausführungsform ist der Aufnahmefinger so ausgebildet, dass der zweite Innenzylinder beim Aufstecken auf den Aufnahmefinger aufgeweitet wird.

In einer weiteren besonderen Ausführungsform ist der Aufnahmefinger so ausgestaltet, dass der zweite Innenzylinder mittels Haftreibung auf dem Aufnahmefinger gehalten wird.

Besonders bevorzugt weist der zweite Innenzylinder einen Aussenflansch auf, der mit dem ersten Innenzylinder in eine gelagerte Fügung eingeht, wenn der erste Innenzylinder über den zweiten Innenzylinder auf den Aufnahmefinger gestülpt wird.

In einer besonderen Ausführungsform ist der Aufnahmefinger erwärmbar ausgebildet. Besonders bevorzugt kann der Aufnahmefinger auf eine Temperatur geheizt werden, die eine Umformung des Materials der Applikator-Teile ermöglicht. So kann insbesondere der Aufnahmefinger eine Temperatur von zwischen 80 und 180° einnehmen und dadurch ein Umformen der Applikator-Teile ermöglichen, wenn diese aufgesteckt werden und ein äusserer Druck appliziert wird. Besonders bevorzugt sind Bearbeitungsstationen vorgesehen, die diesen äusseren Druck aufbringen.

In einer alternativen und/oder ergänzenden Ausführungsform werden die Applikator-Teile ohne Umformen gefügt. Dazu können die Applikator-Teile, insbesondere der Aussenzylinder und der erste Innenzylinder, mit einem Rastmechanismus ausgestatte sein, der ein rein mechanisches Fügen ermöglicht. Die Bearbeitungsstationen können in dieser Ausführungsform zum Beispiel ausgestaltet sein, um eine kinetische Kraft auf ein proximales Ende der Zylinder auszuüben, insbesondere eine kinetische Kraft durch welche der Aussenzylinder und ein erster Innenzylinder und/oder ein erster Innenzylinder und ein zweiter Innenzylinder eine Rastverbindung eingehen können. Diese Vorrichtung kann besonders gut geeignet sein, um Applikatoren zu fügen, die im Wesentlichen aus nicht thermoplastischen Materialien bestehen, z.B. Karton umfassen, oder aus Karton bestehen.

In einer alternativen und/oder ergänzenden Ausführungsform ist die Bearbeitungsstation, respektive sind die Bearbeitungsstationen ausgelegt, den entsprechenden Temperaturbereich von zwischen 80 bis 180° Celsius einzustellen und die entsprechende Umformung durchzuführen. Ein Fachmann kann zum Beispiel den Temperaturbereich in Abhängigkeit der Materialeigenschaften des umzuformenden Innenzylinders einstellen.

In einer besonderen Ausführungsform umfasst der mindestens eine Aufnahmefinger mindestens einen Klemmdornen zur Halterung eines Aussenzylinders und/oder eines ersten Innenzylinders.

Insbesondere kann ein Klemmdornen mit einer Rückhaltekraft ausgebildet sein, welche auf den entsprechenden Zylinder einwirkt und diesen an Stelle hält. Besonders bevorzugt ist der Klemmdornen bewegbar und um eine Achse auf dem Aufnahmefinger schwenkbar oder gefedert ausgestaltet.

Der Klemmdornen kann z.B. ausgestaltet sein, um bei der Aufnahme eines Zylinders eingeklappt zu werden, sodass der Klemmdornen keinen Widerstand gegen die Aufnahme des Zylinders bildet. Ist der Zylinder aufgenommen, so kann der Dornen z.B. über eine gefederte Rückhaltekraft auf den Zylinder wirken und diesen anstelle halten.

In dieser besonderen Ausführungsform kann für einen Ausstoss der Klemmdornen erneut aktiviert werden, sodass er eingeklappt ist und im Wesentlichen mit dem Aufnahmefinger bündig ist, sodass ein Abstreifen des fertiggefügten Tampon-Applikators nicht verhindert wird.

In einer alternativen oder ergänzenden Ausführungsform ist der Klemmdorn als Bügel ausgebildet, der mit einer Rückstellkraft einen Bestückten Zylinder fixiert.

In einer besonderen Ausführungsform ist die erste Bearbeitungsstation und/oder zweite Bearbeitungsstation ausgelegt, einen an einem Aufnahmefinger aufgenommenen Aussenzylinder, ersten Innenzylinder und/oder zweiten Innenzylinder zu umformen. Besonders bevorzugt ist dieses Umformen ein thermisches Umformen. Ganz besonders bevorzugt wird ein thermoplastisches Material mittels Wärmezufuhr erweicht, sodass noch kein Schmelzprozess stattfindet, das Material insgesamt aber leichter umgeformt werden kann.

In einer besonderen Ausführungsform umfasst die Vorrichtung eine Mehrzahl an Fügestationen, insbesondere zwischen zwei und 24 Fügestationen.

Mit einer Mehrzahl an Fügestationen kann batchweise gleichzeitig eine Bearbeitung von mehreren Chargen erfolgen. So kann z.B. eine Charge bestückt werden, während eine weitere Charge bereits umgeformt wird. Besonders bevorzugt entspricht die Anzahl der Fügestationen den maximal geometrisch möglichen Bearbeitungsstationen und Zufuhren. Ebenfalls denkbar ist, dass einzelne Fügestationen als Reserve vorgesehen sind. Weiter können einzelne Fügestationen keine entsprechende Bearbeitungsstation im Gegenüber aufweisen, wenn z.B. eine Abkühlung stattfinden soll. So kann z.B. nach einem thermischen Umformen zunächst eine Fügestation nach einer Drehung der Kreisscheibe keine entsprechende Bearbeitungsstation und/oder Zufuhr als Gegenstück aufweisen, wenn stattdessen eine Abkühlung des zuvor umgeformten Teils stattfinden muss.

In einer besonderen Ausführungsform sind die Zufuhren und/oder Bearbeitungsstationen radial um die Kreisscheibe aufgebaut, sodass sie einen Winkelbereich einnehmen, indem sie einer Fügestation gegenüberliegend angeordnet sind. Besonders bevorzugt sind die Zufuhren, resp. Bearbeitungsstationen mit Zufuhrelementen, oder Bearbeitungselementen versehen, die im Wesentlichen konzentrisch zu Aufnahmefingern auf den Fügestationen im gleichen Winkelbereich angeordnet sind. So können z.B. Zufuhren die aufzusteckenden Zylinder in einer Anzahl Bahnen zu den Aufnahmefingern führen, die der Anzahl Aufnahmefinger entspricht. So können parallel und gleichzeitig alle Aufnahmefinger einer Fügestation beladen werden. Alternativ und/oder ergänzend können die Aufnahmefinger einer Fügestation auch seriell beladen werden, d.h. mit einer Zufuhr, welche nacheinander die Aufnahmefinger in ihrem Winkelbereich bestückt. Dazu kann die Zufuhr z.B. schwenkbar ausgestaltet sein, sodass ein erstes Bestücken in einer konzentrischen Ausrichtung zu einem Aufnahmefinger eine Schwenkbewegung folgt, die ein erneutes konzentrisches Ausrichten hinsichtlich eines zweiten Aufnahmefingers mit sich bringt.

In einer besonders bevorzugten Ausführungsform umfasst die Vorrichtung zwischen zwei und vierundzwanzig Fügestationen, insbesondere zwischen vier und zwanzig, ganz besonders bevorzugt zwölf Fügestationen.

In einer weiteren besonderen Ausführungsform weist jede Fügestation eine Mehrzahl an Aufnahmefingern zur Aufnahme je eines Aussenzylinders eines ersten Innenzylinders und eines allfällig zweiten Innenzylinders auf.

Besonders bevorzugt weist jede Fügestation eine Anzahl von zwischen zwei und 16 Aufnahmefingern auf, weiter besonders bevorzugt zwischen vier und acht Aufnahmefinger, ganz besonders bevorzugt von vier Aufnahmefingern auf.

In einer besonderen Ausführungsform entsprechen die Zufuhren, resp. auf den Bearbeitungsstationen befindliche Bearbeitungseinheiten der Anzahl an Aufnahmefingern und sind so ausgerichtet, dass wenn die Fügestation im entsprechenden Winkelbereich in Wirkverbindung mit der Bearbeitungsstation, resp. der Zufuhr steht, diese konzentrisch mit den Aufnahmefingern angeordnet sind.

In einer besonderen Ausführungsform sind die Bearbeitungseinheiten als Anpressknöpfe ausgebildet, die mit Druck und Wärme einen entsprechend auf dem Aufnahmefinger aufgenommenen Innenzylinder umformen. Besonders bevorzugt weisen die Anpressknöpfe eine im Wesentlichen konische Aufweitung auf, welche in das proximale Ende eines Innenzylinders zur Umformung geschoben wird.

In einer besonderen Ausführungsform umfassen die Zufuhren mindestens eine Vereinzelungseinheit. Die Vereinzelungseinheit kann dazu dienen, die entsprechenden Zylinder möglichst geordnet an eine Bestückung zu übergeben. Die Bestückung kann als Element der Zufuhr die entsprechenden Zylinder auf die Aufnahmefinger laden. Eine Vereinzelung kann über verschiedene Wege verwirklicht werden. Zum Beispiel können ein Schütteltrichter und entsprechende Band- oder Riemenführungen verwendet werden, die eine korrekte Ausrichtung der Zylinder ermöglichen.

In einer besonderen Ausführungsform weisen die Zufuhren Schieber auf, die geeignet sind, die Zylinder auf die Aufnahmefinger zu laden. Diese Schieber können als Stössel ausgestaltet sein, welche die Zylinder auf die Aufnahmefinger pressen. Die Schieber können auch als Greifer ausgestaltet sein, welche den Umfang der Zylinder fassen und sie über die Aufnahmefinger streifen.

Weisen die Zylinder z.B. einen Flansch auf, so kann der Flansch verwendet werden, um die Zylinder entsprechend auszurichten. In diesem Beispiel würde z.B. ein Riemenantrieb so ausgebildet sein, dass die Zylinder im Wesentlichen durch die Riemen hindurchragen und durch den Flansch zwischen den Riemen gehalten werden. Der Flansch liegt auf zwei beabstandeten Riemen auf. Der Riemenabstand entspricht im Wesentlichen dem Aussendurchmesser eines solchen Zylinders.

Bei Zylindern, welche keinen Flansch aufwiesen, z.B. Aussenzylindern, kann eine alternative Sortiermaschine Verwendung finden, welche die entsprechenden Zylinder vereinzelt. Auch diese Zylinder können mit beabstandeten Riemen geführt werden. Der Riemenabstand entspricht hier allerdings nicht dem Aussendurchmesser der Zylinder, sondern ist geringfügig kleiner, sodass die Zylinder auf den Riemen aufliegen. Eine Vereinzelung kann z.B. mittels Rollen stattfinden. So kann ein Übergangsbereich definiert werden, bei dem die Zylinder mit zwei verschiedenen Geschwindigkeiten gefördert werden, besonders bevorzugt, bei dem die Zylinder im Übergangsbereich von einer ersten auf eine zweite Geschwindigkeit beschleunigt werden. Eine solche Beschleunigung führt dazu, dass allfällig miteinander verbundene Aussenzylinder getrennt werden, indem der erste in Förderrichtung mit der neuen Geschwindigkeit in Berührung kommenden Zylinder vom später Folgenden wegbeschleunigt wird.

Besonders bevorzugt umfasst eine solche Zufuhr ein Riemensystem, welches mittels Rollen angetrieben wird und die Zylinder in Richtung eines Bestückungselements fördert. Diese Rollen können als Anpressrollen zusätzlich mit verschiedenen Geschwindigkeiten laufen, sodass eine Vereinzelung der Zylinder stattfinden kann.

Geeignete Fördersysteme, die mit Riemen Applikatoren fördern, kann der Fachmann der WO 2019/233989 entnehmen.

In einer besonderen Ausführungsform umfasst eine erfindungsgemässe Zufuhr zudem eine Ausrichteeinheit. Eine Ausrichteeinheit kann z.B. Zylinder, welche verschiedenartige Enden aufweisen, wie z.B. Aussenzylinder mit einer blütenblattförmigen Mündung und einem Griffbereich, für die Bestückung korrekt ausrichten. Besonders bevorzugt wird die Ausrichtung durch einen optischen Sensor erfasst.

In einer besonderen Ausführungsform umfasst die Ausrichteeinheit einen Roboter, insbesondere einen Mehr-Achs-Robotern als Ausrichteeinheit.

In einer weiteren besonderen Ausführungsform umfasst die Ausrichteeinheit einen Roboter, welcher die zugeführten Aussenzylinder jeweils so ausrichtet, dass die Mündungsöffnung entgegen der radialen Ausdehnung der Aufnahmefinger zeigt, also die Mündungsöffnung auf den Aufnahmefinger aufgestülpt wird und das distale Ende des Tampon-Applikators zuerst auf den Aufnahmefinger fährt.

In einer besonderen Ausführungsform ist die Kreisscheibe drehbar ausgestaltet, sodass die Fügestation jeweils in Wirkverbindung mit einer radial angeordneten Innenzylinderzufuhr, Aussenzylinderzufuhr, ersten und/oder zweiten Bearbeitungsstation gebracht werden kann.

Neben den geschilderten Riemen kann in einer alternativen oder ergänzenden Ausführungsform auch ein Bandantrieb mit entsprechenden Führungsschienen zur Führung der Zylinder verwendet werden.

In einer besonderen Ausführungsform ist die Bearbeitungsstation integral mit der Zufuhr. In dieser Ausführungsform findet ein Umformen zeitgleich mit dem Bestücken des Aufnahmefingers mit einem entsprechenden Zylinder statt. So kann z.B. ein zweiter Innenzylinder zunächst auf einen Aufnahmefinger aufgetragen werden. Anschliessend wird ein erster Innenzylinder über diesen zweiten Innenzylinder gestülpt und auf den Aufnahmefinger aufgetragen und gleichzeitig ein proximales Ende des zweiten Aufnahmefingers erwärmt, sodass ein Stopp entsteht, der verhindert, dass der erste Innenzylinder wieder vom zweiten Innenzylinder abfällt. Entsprechend analog kann ein Umformen mit dem Beladen jedes Zylinders zeitgleich stattfinden, sodass ein Fügen und ein Bestücken synchron stattfinden.

In einer weiteren besonderen Ausführungsform findet das Bestücken über entsprechende Zufuhren statt, und lediglich eine Bearbeitungsstation führt sämtliche Umformschritte in einem einzelnen Prozess durch. So kann z.B. ein Beladen des Aufnahmefingers dergestalt geschehen, dass noch keine Kraft auf die einzelnen Zylinder ausgeübt wird. Nachdem alle Zylinder auf den Aufnahmefinger aufgetragen werden, wird in einem einzelnen Schritt mittels Zufuhr von Wärme ein Druck ausgeübt, welcher die entsprechend zu verformenden Zylinderteile umformt und die Fügung abschliesst.

Mit der erfindungsgemässen Vorrichtung kann schnell und effizient chargenweise eine Fügung von mehrteiligen Tampon-Applikatoren stattfinden, welche sich platzsparend und effizient realisieren lässt.

Besonders geeignet ist die erfindungsgemässe Vorrichtung zum Fügen teleskopartiger dreiteiliger Tampon-Applikatoren. Diese Tampon-Applikatoren verfügen wie eingangs geschildert über einen Aussenzylinder, welcher den Tampon beherbergt und eine Mündungsöffnung aufweist, durch die der Tampon in die Körperöffnung ausgestossen wird. Weiter weisen diese vorzugsweise einen Griffbereich auf, der sich proximal am Aussenzylinder befindet und einen verjüngten Bereich bildet. Dieser Bereich kann vorzugsweise als Lager zur Führung eines ersten Innenzylinders dienen, der in einem verpackten Zustand fast vollständig in diesem Griffbereich untergebracht ist. Dieser Innenzylinder beherbergt einen zweiten Innenzylinder, der auf analoge Weise von diesem Innenzylinder lagernd geführt ist. Ein Rastmechanismus am proximalen Ende des ersten Innenzylinders führt dazu, dass wenn der zweite Innenzylinder teleskopartig aus dem ersten Innenzylinder gezogen wird, dieser mit dem zweiten Innenzylinder starr verrastet. Wird nun der entstandene Stössel aus diesen beiden Innenzylindern erneut in den Aussenzylinder gedrückt, so wird der Tampon durch die Mündungsöffnung in die Körperöffnung entlassen.

Ein weiterer Aspekt der vorliegenden Erfindung betrifft ein Verfahren zum Fügen mehrteiliger Tampon-Applikatoren, insbesondere eingangs geschilderter dreiteiliger Tampon-Applikatoren. Das Verfahren umfasst den Schritt des Ausrichtens einer Fügestation mit einer ersten Innenzylinderzufuhr. Ein Ausrichten kann z.B. im Sinne der vorliegenden Erfindung und unter Berücksichtigung der oben geschilderten Vorrichtung bedeuten, dass eine Fügestation in einen Winkelbereich einer Zufuhr eintritt und sich im Wesentlichen an dieser Zufuhr ausrichtet, sodass eine Wirkverbindung zwischen der Zufuhr und der Fügestation entstehen kann. Das erfindungsgemässe Verfahren umfasst weiter den Schritt des Bestückens eines ersten Aufnahmefingers der Fügestation mit einem ersten Innenzylinder. Dieses Bestücken kann batchweise erfolgen, d.h. es kann eine beliebige Anzahl an Aufnahmefingern einer Fügestation gleichzeitig mit den Innenzylindern? bestückt werden, oder sie kann seriell erfolgen, indem die Aufnahmefinger von einer Bestückungsstation abgefahren werden und ein Aufnahmefinger nach dem anderen mit einem Innenzylinder versehen wird.

Anschliessend umfasst das erfindungsgemässe Verfahren den weiteren Schritt des Ausrichtens der Fügestation mit einer Aussenzylinderzufuhr oder einer zweiten Innenzylinderzufuhr. Letzteres ist der Fall bei einem z.B. dreiteiligen Tampon-Applikator, wo zunächst ein weiterer Innenzylinder aufgetragen wird, der entsprechend wie oben geschildert z.B. in einem Griffbereich des Aussenzylinders gelagert sein kann.

Das Ausrichten der radial auf einer Kreisscheibe angeordneten Fügestation erfolgt durch eine Kreisrotation der Kreisscheibe. Dies kann z.B. bedeuten, dass die Kreisscheibe um ihren Mittelpunkt in einem bestimmten Winkelbereich verdreht wird. Vorzugsweise erfolgt diese Drehung getaktet. Alternativ und/oder ergänzend kann diese Drehung auch kontinuierlich erfolgen, sofern die Steuerung der entsprechenden Bearbeitungsstationen angepasst ist, z.B. durch Synchronisation mit der Drehung der Kreisscheibe. Auch bei einer kontinuierlichen Drehung gibt es einen Zeitpunkt, indem die Bearbeitungsstationen, resp. die Zufuhren konzentrisch mit den entsprechenden Aufnahmefingern ausgerichtet sind. In diesem Zeitpunkt kann das entsprechende Bearbeiten, resp. Bestücken stattfinden.

Besonders bevorzugt findet das Ausrichten der radial auf einer Kreisscheibe angeordneten Fügestation so statt, dass der Aufnahmefinger in eine koaxiale Wirkverbindung mit der Innenzylinderzufuhr und/oder der Aussenzylinderzufuhr und/oder einer Bearbeitungsstation gebracht wird.

Besonders bevorzugt findet dies bei einer Mehrzahl an Fügestationen für jede Fügestation gleichzeitig statt. Das heisst, zur gleichen Zeit, wie eine Fügestation ihre Aufnahmefinger in konzentrischer Ausrichtung zu einer ersten Zufuhr aufweist, weist eine andere Fügestation ihre Aufnahmefinger konzentrisch hinsichtlich einer zweiten Zufuhr auf. So können bereits bestückte Aufnahmefinger, welche z.B. schon einen zweiten Innenzylinder aufweisen, gleichzeitig mit dem ersten Innenzylinder bestückt werden, während bereits leere und bereite Aufnahmefinger erneut erste Innenzylinder aufnehmen.

In einer besonderen Ausführungsform umfasst das erfindungsgemässe Verfahren weiter das Ausrichten der Fügestation mit einer Bearbeitungsstation. Diese Bearbeitungsstation ist vorzugsweise eine Umformstation. Somit umfasst dieses Verfahren weiter den Schritt des Umformens des ersten Innenzylinders und/oder des zweiten Innenzylinders durch Beaufschlagung mit Wärme und Druck. Wie geschildert, können diese Schritte jeweils synchron ablaufen. Besonders bevorzugt umfassen die Bearbeitungsstationen Bearbeitungseinheiten, zum Beispiel Anpressknöpfe, die in Wirkverbindung mit den Innenzylindern auf den Aufnahmefingern treten können.

In einer besonderen Ausführungsform wird beim Bestücken der Fügestation zunächst der Innenzylinder mit dem kleinsten Durchmesser des mehrteiligen Tampon-Applikators auf den ersten Aufnahmefinger bestückt. Insbesondere wird zunächst der Innenzylinder mit dem kleinsten Durchmesser auf einen Spitzenbereich mit dem geringsten Durchmesser des Aufnahmefingers bestückt.

In einer weiteren besonderen Ausführungsform des erfindungsgemässen Verfahrens wird der Aussenzylinder mittels eines einer Rückstellkraft ausübenden Klemmdorns auf dem Aufnahmefinger verklemmt. Besonders bevorzugt wird er lösbar verklemmt.

Dieses Lösen kann in einer weiteren besonderen Ausführungsform aktuiert über die Steuerung eines am Aufnahmefinger gelagerten Drehpunktes stattfinden. Dazu wird z.B. der Klemmdorn entgegen der Rückstellkraft mit dem Aufnahmefinger bündig gemacht, sodass dieser keinen Widerstand gegen den Ausstoss des fertiggefügten Tampon-Applikators aufbringt.

In einer alternativen Ausführungsform wird der Aufnahmezylinder entgegen der Rückstellkraft eines Klemmdorns auf den Aufnahmefinger aufgetragen, sodass bei Lösen einer Arretierung diese Rückstellkraft einen Ausstoss des fertigen Tampon-Applikators erzeugt.

Für einen Fachmann versteht es sich von selbst, dass alle genannten Ausführungsformen in einer erfindungsgemässen Verwirklichung beliebig miteinander kombiniert auftreten können, sofern sie sich nicht gegenseitig ausschliessen.

Im Folgenden wird die vorliegende Erfindung nun anhand konkreter Beispiele und Figuren näher erläutert, ohne auf diese beschränkt zu sein.

Ein Fachmann kann aus diesen Beispielen weitere vorteilhafte Ausführungsformen für die Ausführung der vorliegenden Erfindung entnehmen.

Die Figuren sind schematisch, und der Einfachheit halber wurden die gleichen Teile mit den gleichen Bezugszeichen versehen.

### Fig urenbeschrieb

Es zeigen:
- Fig. 1a: eine Kreisscheibe mit einer Mehrzahl an Fügestationen;
- Fig. 1b: die Kreisscheibe der Fig. 1a in Aufsicht;
- Fig. 2a: exemplarisch eine mit einer Bearbeitungsstation ausgerichteten Fügestation;
- Fig. 2b: die ausgerichtete Fügestation der Fig. 2a im Detail;

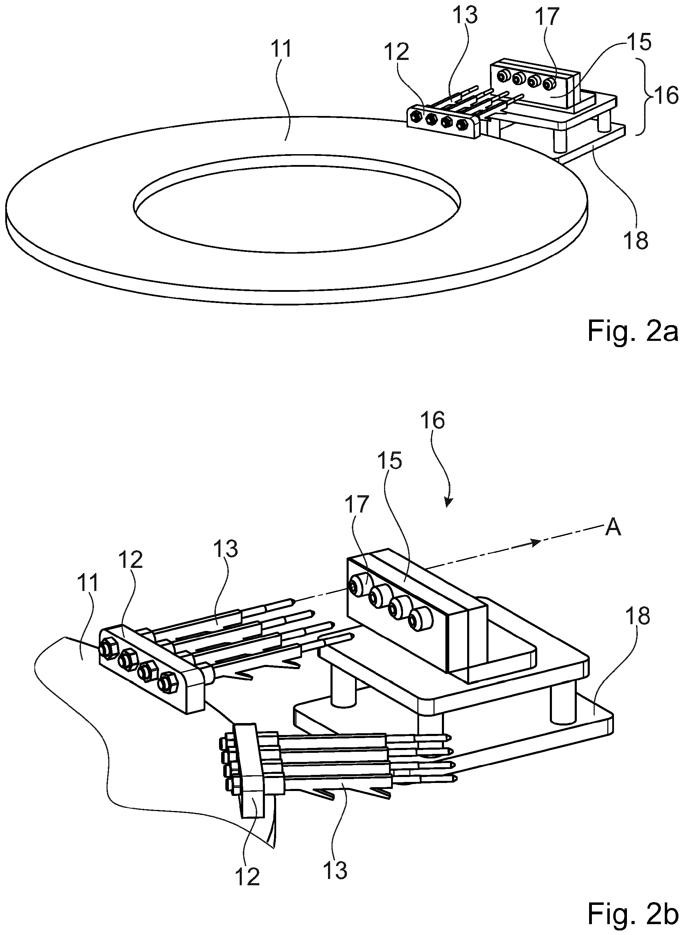

- Fig. 2b: die Fügestation der Fig. 2a in Aufsicht;

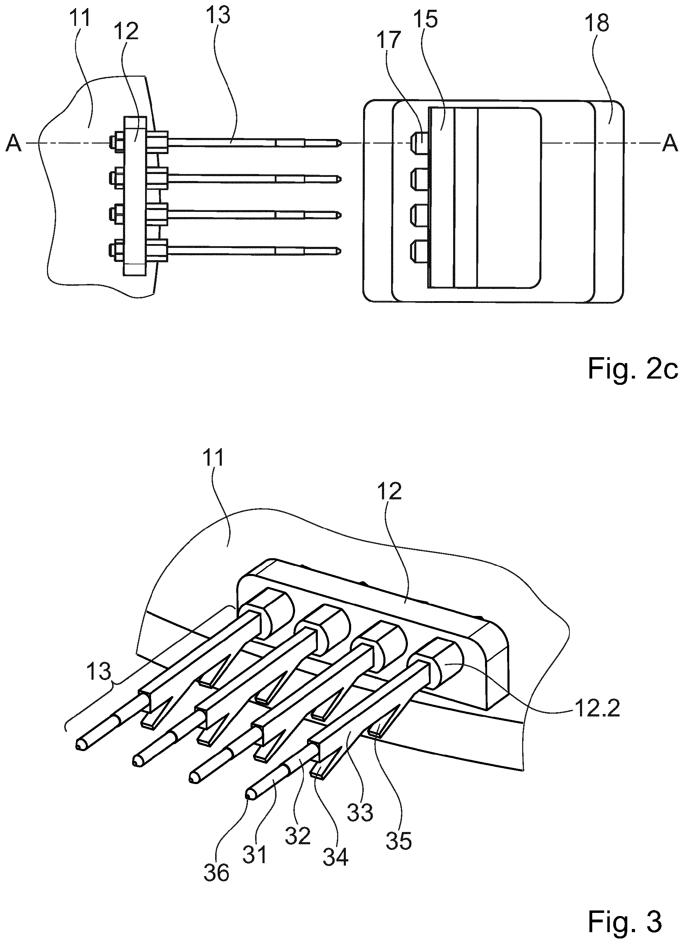

- Fig. 3: eine erfindungsgemässe Fügestation in Frontalansicht;

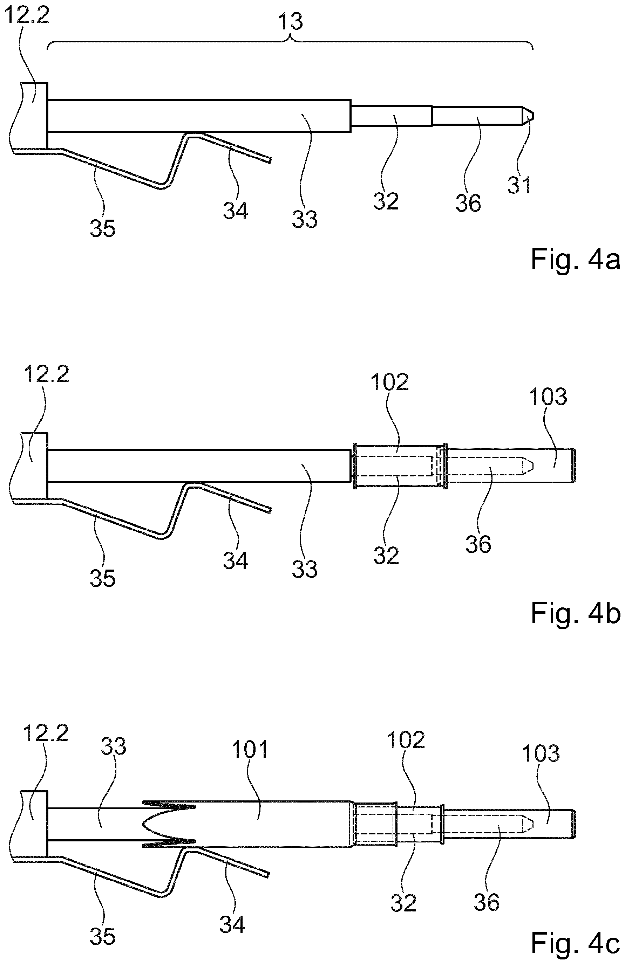

- Fig. 4a: eine Profilansicht eines Aufnahmefingers;
- Fig. 4b: der Aufnahmefinger der Fig. 4a bestückt mit zwei Innenzylindern;
- Fig. 4c: der Aufnahmefinger der Fig. 4a bestückt mit allen Teilen eines dreiteiligen Tampon-Applikators;

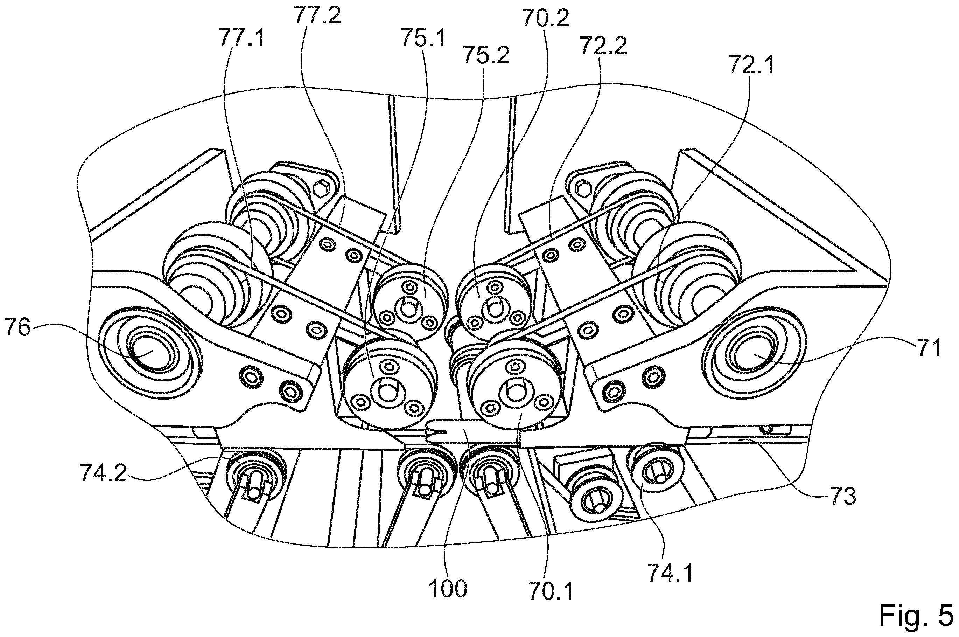

- Fig. 5: eine Vereinzelungseinheit;

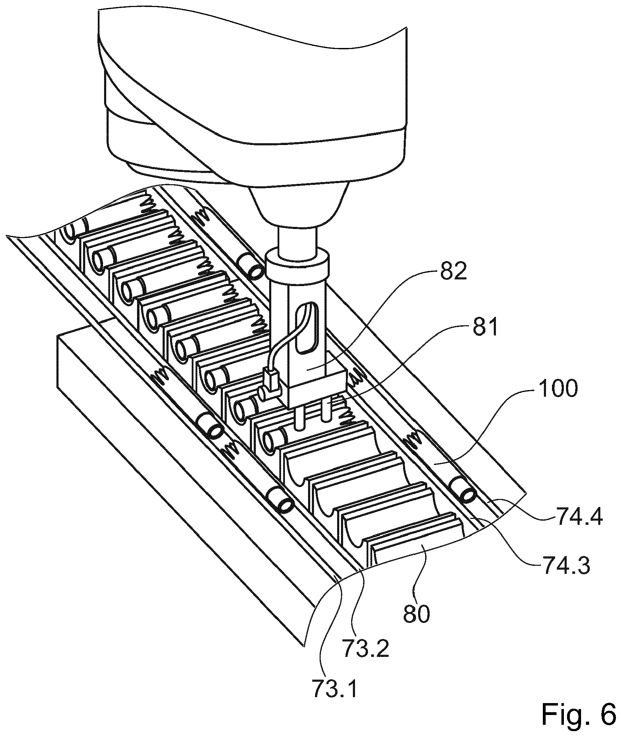

- Fig. 6: eine Ausrichtungseinheit, und

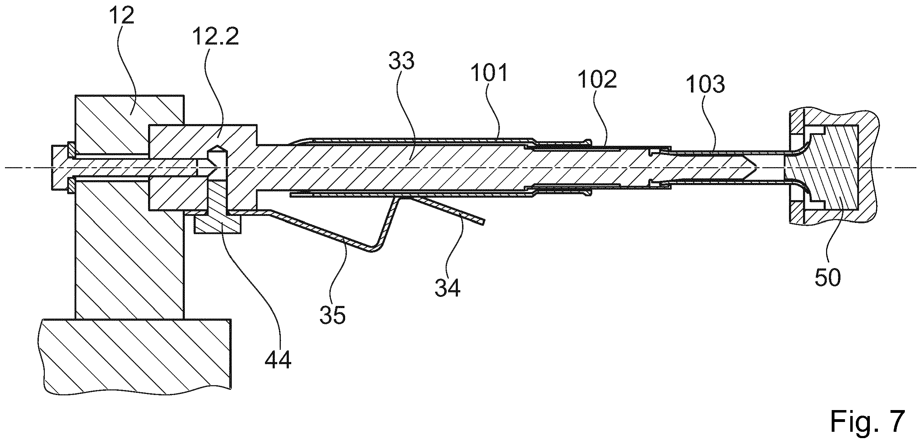

- Fig. 7: eine Bearbeitungsstation.

### Ausführung der Erfindung

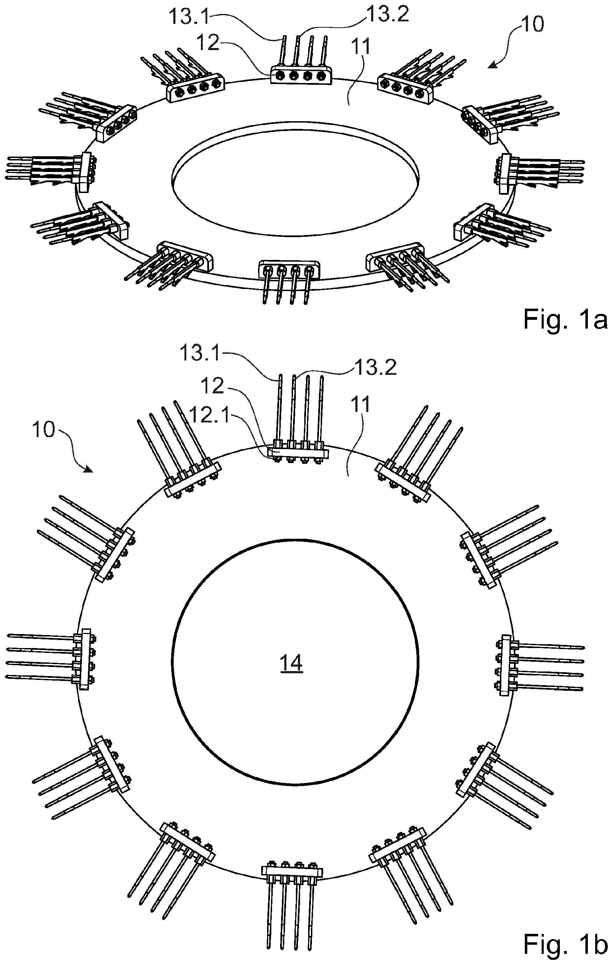

Die Fig. 1a zeigt schematisch eine Kreisscheibe 11. Die Kreisscheibe 11 ist als ringförmige Scheibe ausgebildet und das zentrale Element der erfindungsgemässen Vorrichtung. Im vorliegenden Beispiel weist die Kreisscheibe 11 eine Mehrzahl an Fügestationen 12 auf. Konkret sind es in diesem Beispiel zwölf Fügestationen 12. Die Fügestationen 12 sind auf der Kreisscheibe 11 radial angeordnet im Wesentlichen entlang des Aussenumfangs der Kreisscheibe 11. Im vorliegenden Beispiel zeigt die Kreisscheibe 11 eine zentrale Ausnehmung, dies ist allerdings nicht erforderlich. Bevorzugt ist die Kreisscheibe 11 drehbar angetrieben. Dies kann z.B. mittels eines Zahngetriebes auf der Unterseite der Kreisscheibe 11 (nicht gezeigt) erfolgen. Ebenfalls denkbar wäre ein Antrieb mittels eines Riemens, ein elektrischer Antrieb oder ein Kreismotor mit entsprechender Wirkverbindung zwischen Achse und Kreisscheibe 11.

Die Fügestationen 12 weisen eine Mehrzahl an Aufnahmefingern auf. Im vorliegenden Beispiel weist die Fügestation 12 insgesamt vier Aufnahmefinger 13.1, 13.2 auf. Die Aufnahmefinger 13.1, 13.2 erstrecken sich im Wesentlichen radial vom Mittelpunkt der Kreisscheibe aus deren Aussenumfang. Dadurch können die Fügestationen 12 und die entsprechenden Aufnahmefinger 13.1, 13.2 in Wirkverbindung gebracht werden mit aussen am Umfang ebenfalls radial vorgesehenen Bearbeitungseinheiten, resp. Zufuhreinheiten. Die entsprechende Ausstattung, d.h. die Anzahl an Fügestationen und die entsprechende Anzahl an Aufnahmefingern sind vom Fachmann anpassbar an die geometrischen Verhältnisse und an die entsprechenden verfügbaren Zufuhren, resp. Bearbeitungsstationen. Im vorliegenden Beispiel sind die Aufnahmefinger alle im Wesentlichen auf einer Ebene vorgesehen. Ebenfalls denkbar sind allerdings Fügestationen, welche vertikal versetzte Aufnahmefinger haben oder gar gestaffelte Aufnahmefinger. Solche Fügestationen können z.B. stockwerkweise aufgebaut sein mit einer gewissen Anzahl an Aufnahmefingern auf einer Ebene.

In der Fig. 1b wird ein Stationselement 10 gemäss der Fig. 1a in Aufsicht gezeigt. Die Kreisscheibe 11 weist eine zentrale Ausnehmung 14 auf, welche vornehmlich dazu dient, die bewegte Masse zu reduzieren. Auf der Kreisscheibe 11 sind insgesamt zwölf Fügestationen 12 angebracht, welche einen Fügeblock 12.1 umfassen, aus dem vier Aufnahmefinger 13.1, 13.2 aus dem Aussenumfang der Kreisscheibe 11 hinausragen in einen Bearbeitungsraum. In diesem Bearbeitungsraum können z.B. Zufuhren und Bearbeitungsstationen eingreifen. Diese Zufuhren und Bearbeitungsstationen können einzelne oder eine Mehrzahl an Arbeitsschritten an den Zylindern vornehmen, welche zunächst auf den Aufnahmefingern fixiert werden. Die Bearbeitungsschritte können ein weiteres Bestücken, ein Umformen, ein Prägen, ein Imprägnieren, ein Beschichten, ein Perforieren und/oder ein Ausstossen eines Zylinders umfassen. Dadurch, dass die Bearbeitung auf einem Radius geschieht, kann die vorliegende Station 10 platzsparend eingesetzt werden. In den Bereich der Kreisscheibe 11 können wahlweise Roboter eingreifen, um allfällig notwendige Operationen an den Fügestationen vorzunehmen. Im Bearbeitungsbereich, d.h. direkt am Aussenumfang der Kreisscheibe 11, kann ein Durchfall vorgesehen sein, bei dem z.B. fehlerhaft befundene Zylinder oder Tampon-Applikatoren nach erfolgtem Ausstoss aufgefangen und abgeführt werden können. Alternativ kann dieser Bereich auch zur Aufnahme der fertigen Tampon-Applikatoren dienen, welche nach erfolgter Fügung von den Aufnahmefingern abgestreift werden.

Durch die kompakte Bauweise können optische Sensoren den gesamten Bearbeitungsbereich überblicken. So kann stets eine Kontrolle der Arbeitsschritte stattfinden, und es ist möglich, frühzeitig einzugreifen, sollte es zu Störungen kommen. Optische Sensoren können an mehreren Stellen auch zur detaillierten Überwachung einzelner Bearbeitungsschritte eingesetzt werden. So können z.B. die abschliessenden Umformungsaktionen mittels optischer Sensoren überwacht werden. So kann z.B. überprüft werden, ob ein innerhalb einer Charge nicht korrekt umgeformter Tampon-Applikator vorhanden ist, und entsprechend aussortiert werden.

In der Fig. 2a ist zur Einfachheit eine Kreisscheibe 11 mit einer einzelnen Fügestation 12 gezeigt. Aus dieser Fügestation 12 ragen vier Aufnahmefinger in einen Bearbeitungsbereich. Diese Fügestation 12 hat sich in den Winkelbereich und somit in die Wirkverbindung mit einer Bearbeitungsstation 16 begeben. Die Bearbeitungsstation 16 verfügt über eine Mehrzahl an Pressen 17, welche konzentrisch mit den Aufnahmefingern ausgerichtet sind. Die Pressen 17 können z.B. ausgerichtet sein, um ein Umformen eines proximalen Endes eines Tampon-Applikators herbeizuführen. Ein entsprechend am äussersten und kleinsten Durchmesser des Aufnahmefingers 13 aufgesteckter zweiter Innenzylinder könnte z.B. mittels einer entsprechenden Gegenform der Anpressknöpfe 17 proximal aufgeweitet und umformt werden, sodass ein natürlicher Stopp entsteht. Die Pressen 17 sind auf einem Block 15 aufgebracht und ruhen auf einer Platte 18. Die Platte 18 ist entgegen dem Radius der Kreisscheibe 11 bewegbar angeordnet. Alternativ können auch die Fügestationen gegen die entsprechenden Bearbeitungsstationen in radialer Richtung bewegbar sein.

In der Fig. 2b ist diese Anordnung nochmals detaillierter gezeigt. Auf der Kreisscheibe 11 sind zwei Fügestationen 12 dargestellt, welche über eine Mehrzahl, insgesamt im vorliegenden Beispiel vier Aufnahmefingern 13 verfügen. Diesen entgegengesetzt in einer konzentrischen Ausrichtung und im Wesentlichen parallel zur radialen Ausdehnung Ader Kreisscheibe 11 sind Anpressknöpfe 17 auf einer Bearbeitungsplatte 15 einer Bearbeitungsstation 16 angeordnet. Diese Anpressplatte 15 ist bewegbar, sodass sie sich entgegen der radialen Ausdehnung A auf die Aufnahmefinger 13 zubewegen, und entsprechend eine Umformung auf beladene Zylinder ausüben kann.

In der Fig. 2c ist diese Vorrichtung in Aufsicht gezeigt, bei der die Kreisscheibe 11 die besagte Führungsstation 12 aufweist, welche vier sich radial erstreckende Aufnahmefinger 13 hat. Die Längsrichtung der Aufnahmefinger ist konzentrisch mit den Anpressknöpfen 17 auf einer Bearbeitungsplatte 15. Die Bearbeitungsplatte 15 ist auf einer Schiene gelagert, sodass sie auf einer Feststellplatte 18 verschiebbar in Richtung der Aufnahmefinger einen Anpressdruck auf diese erzeugen kann.

In der Fig. 3 ist eine entsprechende Fügestation 12 im Detail gezeigt. Die Fügestation 12 verfügt hier über vier Manschetten 12.2, aus denen jeweils ein Aufnahmefinger 13 hinausragt. Der Aufnahmefinger 13 verfügt über eine Mehrzahl an Durchmessern 31, 32, 33. Insgesamt verfügt der Finger 13, ausgehend von der Manschette, über einen ersten Durchmesser 33. An diesem ersten Durchmesser sind zwei Klemmdornen 35, 34 vorgesehen. Dieser erste Durchmesser 33 ist geeignet, um einen Aussenzylinder aufzunehmen. Die beiden Klemmdornen 33, 34 dienen dazu, mittels einer Rückstellkraft diesen Aussenzylinder zu halten. Im Betrieb wäre der Aussenzylinder im vorliegenden Beispiel der letzte Zylinder, der auf den Aufnahmefinger aufgetragen wird.

An diesen ersten Durchmesser schliesst ein zweiter Durchmesser 32 an, welcher kleiner ist als der Innendurchmesser des ersten Innenzylinders. Im Betrieb wäre dieser erste Innenzylinder der zweite Zylinder, der aufgetragen würde beim vorliegenden Beispiel, welches sich auf dreiteilige Tampon-Applikatoren bezieht.

Direkt an diesen zweiten Durchmesser schliesst ein dritter Durchmesser an, der über eine Schulter sich verjüngt und in eine Spitze 36 mündet. Dieser letzte Durchmesser 31 ist geeignet, einen zweiten Innenzylinder aufzunehmen.

Im Betrieb würde zunächst ein zweiter Aussenzylinder auf diesen letzten Durchmesser 31 aufgestülpt. Durch den sich verjüngenden Bereich, ausgehend vom mittleren Durchmesser 32, wird der zweite Innenzylinder mit dem Aufnahmefinger im Wesentlichen lösbar verklemmt.

Um diese Wirkungsweise zu illustrieren, ist in den Figuren 4a bis 4c diese schrittweise Bestückung gezeigt. Die Figur 4a zeigt einen Aufnahmefinger 13 im Profil. Links an eine Manschette 12.2 würde die Fügestation beginnen. Aus der Manschette 12.2 ragt ein erster Durchmesser 33, welcher zwei als Bügel ausgeformte Klemmdornen 35, 34 aufweist. Die beiden Klemmdornen 35, 34 üben eine Rückstellkraft auf den Aufnahmefinger aus, sodass nach Aufnahme eines Aussenzylinders dieser gehalten wird.

Der erste Durchmesser 33 schliesst mit einem Stopp, auf den ein zweiter Durchmesser 32 folgt. Auf diesem zweiten Durchmesser 32 folgt ein weiterer Stopp und eine sich in den dritten Durchmesser 36 verjüngende Strecke. Abschliessen tut der Aufnahmefinger in einer im Wesentlichen konisch zulaufenden Spitze 31.

In der Fig. 4b sind bereits zwei Innenzylinder 102, 103 auf den Aufnahmefinger aufgetragen. Ein zweiter Innenzylinder 103 wurde zunächst über den letzten Durchmesser aufgestülpt, sodass er am Stopp anschlägt und im sich verjüngenden Bereich verklemmt wird. Über diesen wird ein erster Innenzylinder 102 gestülpt, welcher im Wesentlichen bis zum Ende des zweiten Durchmessers ragt.

In der Fig. 4c wird nun gezeigt, wie ein Aussenzylinder über diese beiden Innenzylinder 102, 103 gestülpt wird.

Die als Bügel ausgeformten Klemmdornen 35, 34 üben eine Rückstellkraft auf den Aussenzylinder 101 aus. Dabei steht ein erstes Bügelteil 35 in einem Winkel von der Manschette 12.2 ab und übt einen Anpressdruck auf den Aussenzylinder 101 aus. Ein zweites Bügelteil 34 ist integral mit dem ersten Bügelteil 35 steht ebenfalls in einem Winkel ab und dient als Lenkblech für die Aufnahme des Aussenzylinders 101.

Die Fig. 5 zeigt eine Vereinzelungseinheit, wie sie verwendet werden kann, um Aussenzylinder für die Zufuhr zu vereinzeln. Es ist wichtig, dass entsprechende Aussenzylinder, welche eine blütenblattförmige distale Mündung aufweisen, voneinander getrennt werden, da sie sonst leicht verhaken und nicht geordnet auf die Aufnahmefinger geladen werden können.

Im vorliegenden Beispiel werden die Zylinder 100 auf Riemen 73 gefördert. Antriebsrollen 74.1, 74.2 treiben die entsprechenden Riemen an. Im gezeigten Beispiel findet eine Übergabe von einem Riemensystem auf das andere statt. Über dieser Übergabestelle sind gummierte Beschleunigungsrollen 75.1, 75.2 vorgesehen, welche ebenfalls mittels eines Riemenantriebs 77.1, 77.2 angetrieben werden. Hier gelangen die Aussenzylinder 100 von einem Antrieb mit einer ersten Geschwindigkeit 70.1, 70.2 in einen Bereich mit einem Antrieb mit einer zweiten Geschwindigkeit 75.1, 75.2. Durch die Beschleunigung werden allfällig ineinander verhakte Aussenzylinder getrennt. Angetrieben werden die Riemen mit unterschiedlicher Geschwindigkeit laufende Antriebswellen 71, 76.

Die Fig. 6 zeigt eine Ausrichtungseinheit, welche ebenfalls die besagten Aussenzylinder 100 für die Beladung der Aufnahmefinger gleich ausrichtet. Wie vorhin wird die Zufuhr der Aussenzylinder 100 über zwei parallellaufende Riemenantriebssysteme mit je zwei Riemen 73.1, 73.2; 74.3, 74.4 bewerkstelligt. Ein Roboter mit einem Roboterarm 82 und einem Greifer 81 greift die Aussenzylinder 100 und überführt sie in eine Förderanlage 80, in der die Aussenzylinder 100 im Wesentlichen parallel und alle gleich ausgerichtet sind. Die Zufuhr endet in einem weiteren Robotersystem (nicht gezeigt), welcher z.B. eine Charge an Aussenzylindern aus dieser Fördereinheit 80 entnehmen kann und entsprechend auf die Aufnahmefinger zur Bestückung dieser lädt.

Die Figur 7 zeigt, wie eine Fügestation 12 mit Aufnahmefinger gerade in Wirkverbindung mit einer Bearbeitungsstation steht. Die Fügestation 12 weist auf einer Manschette 12.2 auf einem ersten Durchmesser 33 einen Außenzylinder 101 auf. In diesem steckt ein erster Innenzylinder 102, in diesem wiederum ein zweiter Innenzylinder 103 steckt.

Der Außenzylinder 101 ist mittels eines federnden, als Bügel ausgeformten Klemmdornen mit einem ersten Bügelteil 35 und einem zweiten Bügelteil 34 gehalten.

Die Bearbeitungsstation weist einen konzentrisch zur Längsachse des Aufnahmefingers ausgebildeten Anpressknopf 50 auf, der sich in das proximale Ende des zweiten Innenzylinders schiebt, und diesen mittels einer konischen Form aufweitet, sodass ein insgesamt trompetenförmiges proximales Ende des zweiten Innenzylinders 103 erzeugt wird.

Durch diese Umformung kann der zweite Innenzylinder 103 nicht aus dem dreiteiligen Tampon-Applikator herausfallen, indem er sich zum Beispiel durch die Mündung des Außenzylinders 101 schiebt. Dieser Schritt kann eine erfindungsgemässe Fügung abschliessen. Dieses Fügen kann aber auch über eine Mehrzahl an einzelnen Teilschritten ablaufen. So kann, zum Beispiel, eine erste Umformung mit einem gewissen ersten Anpressdruck stattfinden und eine zweite Umformung mit einem gewissen zweiten Anpressdruck. Für jede Umformung kann eine eigene Bearbeitungsstation mit jeweiligen Anpressknöpfen bereitgestellt sein. Diese schrittweise Umformung kann so oft wiederholt werden, wie es nötig ist um die gewünschte trompetenförmige Ausweitung des proximalen Endes des Tampon-Applikators zu erhalten. Die erfindungsgemässe Vorrichtung kann verwendet werden, um platzsparend und effizient in einem schnellen Durchsatz Tampon-Applikatoren aus mehreren Hohlzylindern zu fügen. Besonders geeignet ist die erfindungsgemässe Vorrichtung zur Fügung dreiteiliger Tampon-Applikatoren mit einem teleskopartigen Stössel, der ausgezogen und verrastet wird.

## Patentansprüche

1. Vorrichtung zum Fügen mehrteiliger Tampon-Applikatoren, insbesondere dreiteiliger Tampon-Applikatoren, wobei die mehrteiligen Tampon-Applikatoren einen Aussenzylinder (101) und einen ersten Innenzylinder (102) umfassen, die Vorrichtung umfassend:
a. Mindestens eine Fügestation (12) mit mindestens einem Aufnahmefinger (13.1, 13.2) zur Aufnahme je eines Aussenzylinders (101) und eines ersten Innenzylinders (102);
b. Eine erste Innenzylinder Zufuhr zur Bestückung des mindestens einen Aufnahmefingers mit mindestens einem ersten Innenzylinder (102);
c. Eine Aussenzylinder Zufuhr zur Bestückung des mindestens einen Aufnahmefingers (13.1, 13.2) mit mindestens einem Aussenzylinder (101);
d. Eine erste Bearbeitungsstation (16) zur Fügung des mindestens einen Aussenzylinders (101) mit dem mindestens einen ersten Innenzylinder (102), und
**dadurch gekennzeichnet, dass**
die mindestens eine Fügestation (12) radial auf einer Kreisscheibe (11) angeordnet ist, insbesondere so dass der mindestens eine Aufnahmefinger (13.1, 13.2) im Wesentlichen radial ausgerichtet ist.

2. Vorrichtung gemäss Anspruch 1, wobei die mehrteiligen Tampon-Applikatoren einen zweiten Innenzylinder umfassen und die Vorrichtung weiter umfasst:
a. Eine zweite Innenzylinder Zufuhr zur Bestückung des mindestens einen Aufnahmefingers mit mindestens einem zweiten Innenzylinder, und wobei
die erste Bearbeitungsstation zur Fügung des mindestens einen ersten Innenzylinders mit dem mindestens einen zweiten Innenzylinder ausgelegt ist.

3. Vorrichtung gemäss einem der Ansprüche 1 oder 2, wobei der mindestens eine Aufnahmefinger eine Mehrzahl an Durchmessern aufweist, insbesondere wobei der Aufnahmefinger sich in radialer Ausrichtung verjüngende Durchmesser aufweist.

4. Vorrichtung gemäss Anspruch 3, wobei jeder Durchmesser ausgelegt ist um jeweils einen Aussenzylinder, einen ersten Innenzylinder und einen zweiten Innenzylinder aufzunehmen.

5. Vorrichtung gemäss einem der Ansprüche 1 bis 4, wobei der mindestens eine Aufnahmefinger mindestens einen zur Halterung eines Aussenzylinders und/oder eines ersten Innenzylinders aufweist.

6. Vorrichtung gemäss einem der Ansprüche 1 bis 5, wobei die erste Bearbeitungsstation und/oder eine zweite Bearbeitungsstation ausgelegt ist einen an einem Aufnahmefinger aufgenommenen Aussenzylinder, ersten Innenzylinder und/oder zweiten Innenzylinder zu umformen, insbesondere thermisch zu umformen.

7. Vorrichtung gemäss einem der Ansprüche 1 bis 6, wobei die Vorrichtung eine Mehrzahl an Fügestationen umfasst, insbesondere zwischen zwei und vierundzwanzig Fügestationen umfasst.

8. Vorrichtung gemäss einem der Ansprüche 1 bis 7, wobei jede Fügestation eine Mehrzahl an Aufnahmefinger zur Aufnahme je eines Aussenzylinders und eines ersten Innenzylinders, insbesondere zwischen zwei und sechzehn Aufnahmefinger umfasst.

9. Vorrichtung gemäss einem der Ansprüche 1 bis 8, wobei die Aussenzylinder Zufuhr eine Vereinzelungseinheit umfasst.

10. Vorrichtung gemäss einem der Ansprüche 1 bis 9, wobei die Kreisscheibe drehbar ausgestaltet ist, so dass die Fügestation jeweils in Wirkverbindung mit einer radial angeordneten Innenzylinder Zufuhr, Aussenzylinder Zufuhr, ersten Bearbeitungsstation und zweiten Bearbeitungsstation gebracht werden kann.

11. Verfahren zum Fügen mehrteiliger Tampon-Applikatoren, insbesondere dreiteiliger Tampon-Applikatoren, umfassend die Schritte:
a. Ausrichten einer Fügestation mit einer ersten Innenzylinder Zufuhr;
b. Bestücken eines ersten Aufnahmefingers der Fügestation mit einem ersten Innenzylinder;
c. Ausrichten der Fügestation mit einer Aussenzylinder Zufuhr oder einer zweiten Innenzylinder Zufuhr;
d. Bestücken des Aufnahmefingers der Fügestation mit einem Aussenzylinder oder einem zweiten Innenzylinder, und
**Dadurch gekennzeichnet, dass**
Das Ausrichten der radial auf einer Kreisscheibe (11) angeordneten Fügestation (12) durch eine Kreisrotation der Kreisscheibe erfolgt, insbesondere so dass der Aufnahmefinger in eine koaxiale Wirkverbindung mit der Innenzylinder Zufuhr und/oder Aussenzylinder Zufuhr gebracht wird.

12. Verfahren gemäss Anspruch 10, weiter umfassend den Schritt:
a. Ausrichten der Fügestation mit einer Umformstation;
b. Umformen des ersten Innenzylinders und/oder des zweiten Innenzylinders durch Beaufschlagung mit Wärme und Druck.

13. Verfahren gemäss einem der Ansprüche 11 oder 12, wobei beim Bestücken der Fügestation zunächst der Innenzylinder mit dem kleinsten Durchmesser des mehrteiligen Tampon-Applikators auf den ersten Aufnahmefinger bestückt wird, insbesondere auf einen Spitzenbereich mit dem geringsten Durchmesser des Aufnahmefingers bestückt wird.

14. Verfahren gemäss einem der Ansprüche 10 bis 12, wobei der Aussenzylinder mittels eines eine Rückstellkraft ausübenden Klemmdorns auf dem Aufnahmefinger verklemmt wird, insbesondere lösbar verklemmt wird.

15. Verfahren gemäss einem der Ansprüche 10 bis 12 wobei der Aussenzylinder mittels eines eine Rückstellkraft ausübenden Klemmdorns auf dem Aufnahmefinger ausgestossen wird.

## Claims

1. Device for joining multi-part tampon applicators, in particular three-part tampon applicators, wherein the multi-part tampon applicators comprise an outer cylinder (101) and a first inner cylinder (102), the device comprising:
a. at least one joining station (12) with at least one receiving finger (13.1, 13.2) for receiving one outer cylinder (101) and one first inner cylinder (102) each;
b. a first inner cylinder supply for loading the at least one receiving finger with at least one first inner cylinder (102);
c. an outer cylinder supply for loading the at least one receiving finger (13.1, 13.2) with at least one outer cylinder (101);
d. a first processing station (16) for joining the at least one outer cylinder (101) to the at least one first inner cylinder (102), and
**characterized in that**
the at least one joining station (12) is arranged radially on a circular disk (11), in particular in such a way that the at least one receiving finger (13.1, 13.2) is oriented essentially radially.

2. Device according to claim 1, wherein the multi-part tampon applicators comprise a second inner cylinder and the device further comprises:
a. a second inner cylinder supply for loading the at least one receiving finger with at least one second inner cylinder, and wherein
the first processing station is adapted for joining the at least one first inner cylinder to the at least one second inner cylinder.

3. Device according to any one of claims 1 or 2, wherein the at least one receiving finger has a plurality of diameters, in particular wherein the receiving finger has diameters that taper in radial alignment.

4. Device according to claim 3, wherein each diameter is configured to respectively receive an outer cylinder, a first inner cylinder, and a second inner cylinder.

5. Device according to any one of claims 1 to 4, wherein the at least one receiving finger comprises at least one for holding an outer cylinder and/or a first inner cylinder.

6. Device according to any one of claims 1 to 5, wherein the first processing station and/or a second processing station is designed to re-shape, in particular thermally reshape, an outer cylinder, first inner cylinder and/or second inner cylinder received on a receiving finger.

7. Device according to any one of claims 1 to 6, wherein the device comprises a plurality of joining stations, in particular comprising between two and twenty-four joining stations.

8. Device according to any one of claims 1 to 7, wherein each joining station comprises a plurality of receiving fingers each for receiving an outer cylinder and a first inner cylinder, in particular between two and sixteen receiving fingers.

9. Device according to any one of claims 1 to 8, wherein the outer cylinder supply comprises a separation unit.

10. Device according to any one of claims 1 to 9, wherein the circular disc is rotatable so that the joining station can each be brought into operative connection with a radially arranged inner cylinder supply, outer cylinder supply, first processing station and second processing station.

11. Method for joining multi-part tampon applicators, in particular three-part tampon applicators, comprising the steps:
a. aligning a joining station with a first inner cylinder supply;
b. loading a first receiving finger of the joining station with a first inner cylinder;
c. aligning the joining station with an outer cylinder supply or a second inner cylinder supply;
d. loading the receiving finger of the joining station with an outer cylinder or a second inner cylinder, and
**characterized in that**
the alignment of the joining station (12) arranged radially on a circular disk (11) is effected by a circular rotation of the circular disk, in particular so that the receiving finger is brought into a coaxial operative connection with the inner cylinder supply and/or outer cylinder supply.

12. Method according to claim 10, further comprising the step of:
a. aligning the joining station with a forming station;
b. forming the first inner cylinder and/or the second inner cylinder by applying heat and pressure.

13. Method according to any one of claims 11 or 12, wherein when the joining station is loaded, the inner cylinder with the smallest diameter of the multi-part tampon applicator is first loaded onto the first receiving finger, in particular loaded onto a tip region with the smallest diameter of the receiving finger.

14. Method according to any one of claims 10 to 12, wherein the outer cylinder is clamped, in particular releasably clamped, on the receiving finger by means of a clamping mandrel exerting a restoring force.

15. Method according to any one of claims 10 to 12, wherein the outer cylinder is ejected by means of a clamping mandrel exerting a restoring force on the receiving finger.

## Revendications

1. Dispositif pour assembler des applicateurs de tampon en plusieurs parties, en particulier des applicateurs de tampon en trois parties, dans lequel les applicateurs de tampon en plusieurs parties comprennent un cylindre externe (101) et un premier cylindre interne (102), le dispositif comprenant :
a. au moins un poste d'assemblage (12) avec au moins un doigt de réception (13.1, 13.2) pour la réception d'un cylindre externe (101) et d'un premier cylindre interne (102) ;
b. une première alimentation en cylindre interne pour munir l'au moins un doigt de réception d'au moins un premier cylindre interne (102) ;
c. une alimentation en cylindre externe pour munir l'au moins un doigt de réception (13.1, 13.2) d'au moins un cylindre externe (101) ;
d. un premier poste de traitement (16) pour assembler l'au moins un cylindre externe (101) avec l'au moins un premier cylindre interne (102), et
**caractérisé en ce que**
l'au moins un poste d'assemblage (12) est disposé radialement sur un disque circulaire (11), en particulier de sorte que l'au moins un doigt de réception (13.1, 13.2) soit aligné essentiellement radialement.

2. Dispositif selon la revendication 1, dans lequel les applicateurs de tampon en plusieurs parties comprennent un second cylindre interne, et le dispositif comprenant en outre :
a. une seconde alimentation en cylindre interne pour munir l'au moins un doigt de réception d'au moins un second cylindre interne, et dans lequel
le premier poste de traitement est conçu pour assembler l'au moins un premier cylindre interne avec l'au moins un second cylindre interne.

3. Dispositif selon l'une des revendications 1 ou 2, dans lequel l'au moins un doigt de réception présente une pluralité de diamètres, en particulier dans lequel le doigt de réception présente un diamètre qui se rétrécit en alignement radial.

4. Dispositif selon la revendication 3, dans lequel chaque diamètre est conçu pour accueillir un cylindre externe, un premier cylindre interne et un second cylindre interne.

5. Dispositif selon l'une des revendications 1 à 4, dans lequel l'au moins un doigt de réception présente au moins un doigt pour le maintien d'un cylindre externe et/ou d'un premier cylindre interne.

6. Dispositif selon l'une des revendications 1 à 5, dans lequel le premier poste de traitement et/ou un second poste de traitement est conçu pour former, en particulier thermiquement, un cylindre externe, un premier cylindre interne et/ou un second cylindre interne reçus sur un doigt de réception.

7. Dispositif selon l'une des revendications 1 à 6, le dispositif comprenant une pluralité de postes d'assemblage, en particulier entre deux et vingt-quatre postes d'assemblage.

8. Dispositif selon l'une des revendications 1 à 7, dans lequel chaque poste d'assemblage comprend une pluralité de doigts de réception pour recevoir chacun un cylindre externe et un premier cylindre interne, en particulier entre deux et seize doigts de réception.

9. Dispositif selon l'une des revendications 1 à 8, dans lequel l'alimentation en cylindre externe comprend une unité de séparation.

10. Dispositif selon l'une des revendications 1 à 9, dans lequel le disque circulaire est conçu pour pouvoir tourner, de sorte que le poste d'assemblage respectif peut être mis en liaison fonctionnelle avec, disposés radialement, une alimentation en cylindre interne, une alimentation en cylindre externe, un premier poste de traitement et un second poste de traitement.

11. Procédé pour assembler des applicateurs de tampon en plusieurs parties, en particulier des applicateurs de tampon en trois parties, comprenant les étapes consistant à :
a. aligner un poste d'assemblage avec une première alimentation en cylindre interne ;
b. munir d'un premier cylindre interne un premier doigt de réception du poste d'assemblage ;
c. aligner le poste d'assemblage avec une alimentation en cylindre externe ou une seconde alimentation en cylindre interne ;
d. munir d'un cylindre externe ou d'un second cylindre interne le doigt de réception du poste d'assemblage,
**caractérisé en ce que**
le poste d'assemblage (12) disposé radialement sur un disque circulaire (11) est aligné par une rotation circulaire du disque circulaire, en particulier de sorte que le doigt de réception est amené en liaison fonctionnelle coaxiale avec l'alimentation en cylindre interne et/ou l'alimentation en cylindre externe.

12. Procédé selon la revendication 10, comprenant en outre l'étape consistant à :
a. aligner le poste d'assemblage avec un poste de formage ;
b. former le premier cylindre interne et/ou le second cylindre interne par application de chaleur et de pression.

13. Procédé selon l'une des revendications 11 ou 12, dans lequel, lors de l'étape consistant à munir le poste d'assemblage, le premier doigt de réception, en particulier une zone de pointe de plus petit diamètre du doigt de réception, est d'abord muni du cylindre interne de plus petit diamètre de l'applicateur de tampon en plusieurs parties.

14. Procédé selon l'une des revendications 10 à 12, dans lequel le cylindre externe est serré, en particulier de manière amovible, sur le doigt de réception au moyen d'un mandrin de serrage qui exerce une force de rappel.

15. Procédé selon l'une des revendications 10 à 12, dans lequel le cylindre externe est éjecté du doigt de réception au moyen d'un mandrin de serrage qui exerce une force de rappel.
